# EUROPÄISCHE PATENTANMELDUNG

(11) **EP 2 716 253 A1**
(43) Veröffentlichungstag der Anmeldung: **09.04.2014**
(21) Anmeldenummer: 13187532.0
(22) Anmeldetag: 07.10.2013
(51) Int. Cl.: A61B 19/00, G01S 3/786, G01S 5/16, G06T 7/00, G06T 7/20

(54) **Verfahren zum Steuern eines Telemanipulationsroboters**

(30) Priorität: 05.10.2012 DE 102012218213
(71) Anmelder: Deutsches Zentrum für Luft- und Raumfahrt e.V., 51147 Köln (DE)
(72) Erfinder: Tobergte, Andreas, 80992 München (DE); Passig, Georg, 85092 Köching (DE); Steidle, Florian, 80339 München (DE)
(74) Vertreter: von Kreisler Selting Werner

(57) **Zusammenfassung**

Die Erfindung betrifft ein Verfahren zum Steuern eines Telemanipulationsroboters (10), wobei eine durch eine Person hervorgerufene Bewegung einer Eingabevorrichtung durch mindestens eine Kamera erfasst wird. Hierbei weist die Eingabevorrichtung mindestens drei aktive Marker auf. Es wird eine lokale rekursive Markeridentifikation durchgeführt, indem in mindestens einem Bild der hierauf sichtbare Marker A einem Marker A zugeordnet wird, der auf einem Bild sichtbar ist, das zu einem früheren Zeitpunkt von derselben Kamera aufgenommen wurde. Es wird eine globale Markeridentifikation durchgeführt. Wenn ein Marker A in mindestens zwei Bildern lokal rekursiv identifiziert wurde und in mindestens einem dieser Bilder global nicht rekursiv identifiziert wurde, wird eine globale rekursive Identifikation durchgeführt. Der Marker A gilt dann für alle Bilder, in denen er lokal rekursiv identifiziert wurde als global rekursiv identifiziert.

## Beschreibung

Die Erfindung betrifft ein Verfahren zum Steuern eines Telemanipulationsroboters.

Telemanipulationsroboter können beispielsweise in der Telechirurgie eingesetzt werden, um komplizierte Operationen an einem Patienten durchzuführen. Hierzu ist es möglich, dass der Roboter durch eine Bedienperson, in diesem Fall durch einen Chirurgen unter Verwendung einer Eingabevorrichtung gesteuert wird. Durch eine solche Eingabevorrichtung wird die gewünschte Bewegung des Roboters durch die Bedienperson direkt vorgegeben. Die Bedienperson bedient hierzu ein Eingabegerät, wie z. B. ein haptisches Eingabegerät oder eine Space-Mouse, um Sollbewegungen für den Roboter bzw. die Werkzeugspitze in sechs Freiheitsgraden zu generieren.

Eine Alternative zu Eingabegeräten, welche mechanisch fest mit der Erde, bzw. einer Operatorkonsole verbunden sind, stellen frei im Raum bewegliche Eingabegeräte dar. Zur Bewegungserfassung in allen Raumfreiheitsgraden können optische Sensoren (d.h. Kameras) mit aktiven Markern verwendet werden. Aufgrund der Bewegungen, vor allem der Rotationen, werden einzelne Marker aus dem Sichtbarkeitsbereich einzelner Kameras heraus und an anderer Stelle wieder hinein bewegt. Ein Hauptproblem stellt dabei die schnelle und sichere, d.h. eindeutige Identifikation, der optischen Marker dar.

Aufgabe der Erfindung ist es, ein einfaches und sicheres Verfahren zum Steuern eines Telemanipulationsroboters bereitzustellen.

Die Lösung der Aufgabe erfolgt erfindungsgemäß durch die Merkmale des Anspruchs 1.

Das erfindungsgemäße Verfahren zum Steuern eines Telemanipulationsroboters umfasst die folgenden Verfahrensschritte:
a) Zunächst wird eine durch eine Person hervorgerufene Bewegung einer Eingabevorrichtung durch eine Kamera optisch erfasst. Bevorzugt wird hierbei lediglich eine Kamera verwendet, so dass ein zweidimensionales Bild der erfassten Eingabevorrichtung entsteht. Die Eingabevorrichtung weist mindestens drei und bevorzugt vier oder mehr aktive Marker auf. Unter einem aktiven Marker wird ein Marker verstanden, der aktiv an- und ausgeschaltet werden kann. Hierbei kann es sich beispielsweise um einen Infrarot-LED-Marker handeln. Es ist bevorzugt, dass die Leuchtdauer und/ oder die Leuchtintensität und/ oder die Farbe des aktiven Markers veränderbar ist, um ihn von anderen aktiven Markern, die gleichzeitig leuchten, unterscheiden zu können.
b) Aus den gemäß Verfahrensschritt a) erfassten Bewegungen, der durch die Person bewegten Eingabevorrichtung werden Bewegungsbefehle für den Telemanipulationsroboter berechnet. Hierzu können die Positionen der Marker im Raum, in drei Freiheitsgraden bestimmt werden. Aus der Position der Marker kann die Position und Orientierung (d. h. die Lage) der Eingabevorrichtung berechnet werden. Es ist auch möglich, die Position und Orientierung der Eingabevorrichtung im Rahmen einer 6D-Lageschätzung direkt aus den zweidimensionalen Kamerakoordinaten abzuleiten, ohne dass die dreidimensionale Position der Marker im Raum explizit bestimmt wird. Es ist bekannt, an welchen Stellen der Eingabevorrichtung die Marker befestigt sind (d.h. die Geometrie der Marker in Relation zueinander und zur Eingabevorrichtung ist bekannt).
c) Die berechneten Bewegungsbefehle werden an den Telemanipulationsroboter übertragen, der gemäß den übertragenen Befehlen bewegt wird.

Erfindungsgemäß wird eine lokale rekursive Markeridentifikation durchgeführt, indem in mindestens einem Bild der hierauf sichtbare Marker einem Marker zugeordnet wird, der auf einem Bild sichtbar ist, das zu einem früheren Zeitpunkt von derselben Kamera aufgenommen wurde. Hierbei muss es sich nicht um das unmittelbar vorangehende Bild handeln. Anders ausgedrückt, ist es möglich, dass eine lokale rekursive Markeridentifikation lediglich in jedem zweiten, jedem dritten usw. Bild durchgeführt wird und die dazwischenliegenden Bilder der Kamera für andere Zwecke verwendet werden.

Durch die lokale rekursive Markeridentifikation wird die Frage beantwortet, ob der im aktuellen Bild sichtbare Marker, der Marker ist, der im älteren Bild sichtbar war. Hierzu wird ein Tracking über die Historie der Bewegung des zu identifizierenden Markers durchgeführt. Hierbei wird auf Basis der bis zu diesem Zeitpunkt vorhandenen Bilder für diesen Marker, die weitere Bewegung dieses Markers mit einem Streuradius geschätzt. Der Streuradius ist nötig, um mögliche Beschleunigungen und Richtungsänderungen durch die Bedienperson zu erlauben und ferner um Bildrauschen und Kalibrierfehler zu berücksichtigen. Wenn ein Marker im aktuell untersuchten Bild innerhalb dieses Bereiches, d.h. dieses Streuradius gefunden wird, wird angenommen, dass es der Marker aus den vorangegangenen Bildern ist. Sobald sich mehr als ein Marker innerhalb dieses Streuradius befindet, ist es nicht mehr möglich, einen der Marker lokal rekursiv zu identifizieren. Die Frequenz, mit der die lokal rekursive Identifikation durchgeführt wird, hängt von der zu erfassenden Dynamik und Geschwindigkeit der Bewegung der Eingabevorrichtung ab. Sofern eine Kamera verwendet wird, die eine höhere Bildfrequenz bereitstellt, können Zwischenbilder für die im Folgenden beschriebene global nicht-rekursive Identifizierung verwendet werden.

Erfindungsgemäß erfolgt weiterhin eine globale Markeridentifikation, indem ein Marker global nicht-rekursiv in einem Bild einer Kamera eindeutig identifiziert wird und/ oder dieser Marker alternativ oder zusätzlich durch ein eindeutiges Ansteuerungsmuster in mehreren zeitlich hintereinander folgenden Bildern derselben Kamera, die ebenfalls nicht direkt aufeinanderfolgen müssen, global rekursiv identifiziert wird.

Unter einer globalen nicht-rekursiven Identifikation wird verstanden, dass ein Marker derart aktiviert wird, dass er in einem einzigen Bild von allen anderen Markern unterschieden werden kann. Dies kann dadurch erfolgen, dass dieser Marker in diesem Bild als einziger aktiviert wird, so dass keine anderen Marker auf diesem Bild sichtbar sind. Alternativ ist es möglich, die Leuchtdauer, Leuchtintensität und/ oder Farbe dieses Markers derart zu wählen, dass sie sich von allen anderen auf diesem Bild sichtbaren Markern unterscheidet, so dass hierdurch ebenfalls eine global nicht-rekursive Identifikation dieses Markers möglich ist. Es ist auch möglich, einen Marker dadurch global nicht-rekursiv zu identifizieren, dass alle anderen auf dem aktuellen Bild sichtbaren Marker lokal rekursiv identifiziert sind, so dass lediglich ein einziger Marker übrig bleibt, der somit global rekursiv nicht identifizierbar ist.

Anschließend wird eine globale rekursive Identifikation durchgeführt, wenn ein Marker in mindestens zwei Bildern lokal rekursiv identifiziert wurde und er in mindestens einem dieser Bilder global nicht-rekursiv identifiziert wurde. Dieser Marker gilt dann für alle Bilder, in denen er ohne Unterbrechung lokal rekursiv identifiziert wurde (auch vor der globalen nicht-rekursiven Identifikation) als global rekursiv identifiziert.

Alternativ oder zusätzlich ist es möglich, direkt zu einer global rekursiven Identifizierung eines Markers zu gelangen, ohne dass eine globale nicht-rekursive Identifizierung dieses Markers notwendig ist. Hierzu kann dieser Marker in mehreren zeitlich hintereinander folgenden Bildern durch ein eindeutiges Ansteuerungsmuster aktiviert werden. Bei diesem Ansteuerungsmuster kann es sich um eine vorher festgelegte Variation der Leuchtdauer, Leuchtintensität und/ oder der Farbe dieses Markers handeln, die dem Rechner, der die aufgenommenen Kamerabilder analysiert, bekannt ist. Es handelt sich somit hierbei vorzugsweise um einen Code, der beiden Seiten bekannt ist und der es ermöglicht, den Marker direkt global rekursiv zu identifizieren, sobald eine vorbestimmte Anzahl an Bildern dieses Markers vorliegt. Beispielsweise ist es möglich, eine Art Morsecode zu verwenden, in dem die Leuchtdauer des Markers zwischen kurz und lang variiert wird, so dass über zwei, drei oder mehr Bilder hinweg eine eindeutige Identifizierung (global rekursiv) möglich ist.

Das erfindungsgemäße Verfahren nutzt die Tatsache, dass es nicht notwendig ist, einen Marker in jedem Bild global nicht rekursiv zu identifizieren. Vielmehr ist ausreichend, dies einmal zu tun, sofern dieser Marker anschließend lokal rekursiv getrackt werden kann. Weiterhin kann sich die global rekursive Identifizierung dieses Markers auch auf Bilder erstrecken, die zeitlich vor dem Bild liegen, in dem der Marker global nicht rekursiv identifiziert wurde, sofern auch in diesen vorangegangenen Bildern eine lokale rekursive Identifizierung ohne Unterbrechung möglich war. Eine globale nicht-rekursive Identifizierung durch eine individuelle Ansteuerung des Markers ist z. B. nur dann nötig, wenn der Marker aufgrund einer bestimmten Bewegung der Eingabevorrichtung, die durch die Bedienperson hervorgerufen wurde, aus dem Bildbereich verschwindet. Beispielsweise kann es vorkommen, dass die Bedienperson die Eingabevorrichtung derart dreht, dass ein bestimmter Marker aus dem Sichtbereich verschwindet. Sobald dieser Marker wieder im Sichtbereich erscheint, ist es notwendig eine globale Identifizierung durchzuführen. Existieren zu diesem Zeitpunkt mehrere Marker, die global nicht rekursiv identifiziert werden müssen, kann es vorkommen, dass einer oder mehrere dieser Marker erst in späteren Bildern global nicht-rekursiv identifiziert werden.

Das erfindungsgemäße Verfahren arbeitet somit nicht streng sequenziell, indem jeweils nur ein Marker während einer Belichtungsphase der Kamera aktiviert wird. Das erfindungsgemäße Verfahren arbeitet jedoch auch nicht streng parallel, so dass immer alle Marker aktiviert werden. Vielmehr ist es möglich, abhängig von den durchgeführten Bewegungen der Eingabevorrichtung dynamisch zu entscheiden, welche Marker im aktuellen Bild auf welche Weise aktiviert werden müssen, um eine bestmögliche Identifizierung der Marker durchzuführen, ohne Ressourcen zu verschwenden.

Gegenüber optischen Messsystemen, bei welchen aktive Marker streng sequenziell angesteuert werden, bietet das erfindungsgemäße Verfahren eine höhere Anzahl identifizierter Markermessungen, da die Anzahl der Bilder, in denen jeder Marker sichtbar ist, höher ist. Bei einer streng sequentiellen Aktivierung von Markern, sinkt die Anzahl der Messungen pro Marker proportional zur Gesamtzahl der verwendeten Marker. Insbesondere beim Tracking eines zweihändigen Eingabegeräts, bei dem insgesamt unter Umständen acht oder mehr Marker notwendig sind, ist dies ein erheblicher Nachteil.

Andererseits ist das parallele Aktivieren von allen Markern in allen Messungen nachteilig, da die Zuordnung der Marker zunächst nicht eindeutig ist. Eine Identifikation ist lediglich möglich über die Geometrie und die Historie der vorangegangenen Messungen. Derartige Verfahren sind fehleranfällig, weil sie eine hohe Messgüte erfordern, um eine korrekte Markeridentifikation durchzuführen. Insbesondere wenn die Distanz zwischen zwei Markern gering ist oder sie sich gegenseitig teilweise verdecken, können Mehrdeutigkeiten in der Markergeometrie entstehen. Im Allgemeinen kann eine Identifikation der Marker über die Geometrie nur im dreidimensionalen Raum durchgeführt werden, weil in einem zweidimensionalen Bild die Abstände nicht eindeutig sind. Dies erfordert, dass jeder Marker von mindestens zwei Kameras gesehen werden muss und eine explizite 3D-Rekonstruktion vor der Markeridentifikation durchgeführt wird. Das erfindungsgemäße Verfahren kann dagegen auch basierend auf zweidimensionalen Bildern einer einzigen Kamera durchgeführt werden.

Es ist bevorzugt, dass im Rahmen des erfindungsgemäßen Verfahrens keine dreidimensionale Rekonstruktion der Markerpositionen unter Verwendung von zwei oder mehr Kameras durchgeführt wird. Bei derartigen Verfahren ist es notwendig, dass der gleiche Marker zur gleichen Zeit von mindestens zwei Kameras gesehen werden kann. Ferner sind bei derartigen Verfahren die Kameras meistens parallel zueinander ausgerichtet. Eine gleichzeitige Sichtbarkeit des gleichen Markers durch mindestens zwei Kameras ist beim erfindungsgemäßen Verfahren nicht notwendig. Vielmehr ist es beispielsweise möglich, die Kameras phasenversetzt zu belichten, so dass jede ein individuelles Aktivierungsmuster der Marker aufnimmt. Zusätzliche Kameras werden beim erfindungsgemäßen Verfahren vorzugsweise dazu verwendet, Abschattungen von Markern zu verhindern. Hierdurch wird die Anzahl der Marker erhöht, die durch irgendeine Kamera sichtbar sind. Ferner kann durch das Vorsehen zusätzlicher Kameras das Rauschverhalten in verschiedenen Raumachsen verbessert werden.

Bei der Person, die die Eingabevorrichtung bewegt, handelt es sich vorzugsweise um eine Bedienperson, die in bewusster Weise eine Bewegung der Eingabevorrichtung verursacht, um den Telemanipulationsroboter dementsprechend zu steuern. Alternativ kann jedoch die Eingabevorrichtung in einer Weise mit der Person mechanisch verbunden sein, z. B. indem sie an dieser Person befestigt ist, dass die Person in unbewusster Weise die Eingabevorrichtung bewegt und somit Bewegungsbefehle für den Telemanipulationsroboter erzeugt. Dies kann beispielsweise im Rahmen von Operationen hilfreich sein, bei denen das erfindungsgemäße Verfahren zur Kompensation von Bewegungen verwendet werden kann, die am Operationssitus entstehen. Soll beispielsweise eine Osteotomie durchgeführt werden, so ist es möglich Marker an dem zu trennenden Knochen anzubringen, so dass eine Bewegung dieses Knochens während der Operation automatisch zu einer Bewegung der Marker führt und somit automatisch erfassbar ist. Der Telemanipulationsroboter, der die Operation ausführt, kann in diesem Fall automatisiert die erfassten Bewegungen im Operationssitus kompensieren, indem er automatisch diesen Bewegungen nachgeführt wird. Ein ähnliches Verfahren ist beispielsweise bei einer Herzoperation möglich, bei der die Bewegungen des schlagenden Herzens kompensiert werden sollen und der Telemanipulationsroboter, der die Operation durchführt, mit diesen Bewegungen mitgeführt wird.

Es ist bevorzugt, dass eine globale nicht-rekursive Identifikation eines Markers durchgeführt wird, indem seine Leuchtdauer, Leuchtintensität und/ oder Farbe in einem Bild derart gewählt wird, dass sie sich von der Leuchtdauer, Leuchtintensität und/ oder Farbe aller anderen auf diesem Bild sichtbaren Marker unterscheidet. Es ist auch möglich, eine globale nicht rekursive Identifizierung dadurch durchzuführen, dass alle anderen auf dem aktuellen Bild sichtbaren Marker lokal rekursiv identifiziert sind, so dass lediglich ein einziger Marker übrig bleibt, der somit global nicht-rekursiv identifizierbar ist. In einem nächsten Bild kann ein anderer Marke auf dieselbe Weise global nicht-rekursiv identifiziert werden. Bei der Auswertung eines aufgenommenen Bildes besteht bevorzugt Kenntnis darüber, welcher Marker im aktuellen Bild identifiziert werden soll. Durch eine lokale rekursive Identifikation in folgenden oder vorausgegangenen Bildern kann eine global rekursive Identifikation durchgeführt werden. Dieses Verfahren wird als dynamische Markeridentifikation bezeichnet, da hier im Vorhinein nicht festgelegt ist, welcher Marker in welchem Bild auf welche Weise aktiviert wird.

Es ist bevorzugt, einen Marker, der durch seine einzigartige Leuchtdauer, Farbe und/ oder Leuchtintensität in einem Bild global nicht-rekursiv identifiziert wurde, solange wie möglich lokal rekursiv identifiziert wird, so dass sich für diese Zeitdauer eine global rekursive Identifizierung dieses Markers ergibt und die Farbe, Leuchtdauer und/ oder Leuchtintensität dieses Markers erst dann gegenüber allen anderen Markern variiert wird, wenn dieser Marker nicht mehr ohne Unterbrechung lokal rekursiv identifiziert werden kann.

Weiterhin ist es bevorzugt, dass, wenn sich mehrere Marker in einem Bild derart aneinander annähern, dass eine Fortführung des lokalen rekursiven Identifizierens dieser Marker nicht möglich wäre, einer der Marker deaktiviert oder derart angesteuert wird, dass eine Unterscheidung des anderen Markers möglich ist. Eine derartige Situation kann auftreten, wenn sich in einem zweidimensionalen Bild zwei Marker im Streubereich, der für das lokal rekursive Tracking verwendet wird, befinden. Diese Situation würde dazu führen, dass beide Marker nicht mehr lokal rekursiv identifiziert werden können. Insofern ist es bereits von Vorteil, einen dieser Marker auszuschalten, so dass dieser zwar nicht mehr lokal rekursiv identifiziert werden kann, der jeweils andere Marker, jedoch weiterhin lokal rekursiv identifiziert werden kann. Noch vorteilhafter ist es, einen der Marker durch Variation seiner Farbe, Leuchtdauer und/ oder Leuchtintensität gegenüber dem anderen Marker derart zu variieren, dass die Marker unterschieden werden können und somit beide Marker weiter lokal rekursiv identifiziert werden können.

Sofern einer der sich voraussichtlich überschneidenden Marker abgeschaltet werden soll, ist es bevorzugt, bei der Wahl des abzuschaltenden Markers eine Priorisierung vorzunehmen. Beispielsweise kann derjenige Marker abgeschaltet werden, von dem aufgrund der Bewegungshistorie angenommen werden kann, dass er ohnehin nicht mehr über einen langen Zeitraum sichtbar sein wird und der somit in absehbarer Zeit nicht mehr lokal rekursiv identifizierbar ist. Eine Weiterverfolgung dieses Markers wäre somit von geringem Wert. Auch ist es möglich, denjenigen Marker abzuschalten, der aufgrund seiner geometrischen Position weniger zur Verbesserung der Qualität bei der Berechnung der Lage der Eingabevorrichtung beiträgt. Beispielsweise kann ein Marker abgeschaltet werden, der sich relativ nah bei anderen eindeutig identifizierten Markern befindet und der somit einen unwesentlichen Beitrag zur Qualitätsverbesserung leistet. Es ist somit vorteilhafter, denjenigen Marker weiter lokal rekursiv zu identifizieren, der eine größere Entfernung zu anderen eindeutig identifizierten Markern aufweist.

Es ist weiterhin bevorzugt, dass eine globale nicht-rekursive Identifizierung eines Markers durchgeführt wird, indem basierend auf der bekannten Geometrie der Anordnung der Marker, insbesondere unter Einbeziehung von Messwerten einer Inertialmesseinheit mit einem Beschleunigungssensor, Drehratensensor und/ oder einem Kompass eine Hypothese darüber aufgestellt wird, welcher Marker gerade sichtbar ist. Dieser Marker kann anschließend gezielt durch eine Variation seiner Leuchtdauer, Leuchtintensität und/ oder Farbe gegenüber allen anderen in diesem Bild sichtbaren Markern angesteuert werden. Wenn es sich bei diesem Marker tatsächlich um den in der Hypothese vermuteten Marker handelt, ist diese Hypothese verifiziert und der Marker ist somit global nicht-rekursiv identifiziert.

Das im Folgenden beschriebene Verfahren kann als statische Markeridentifikation bezeichnet werden, da es hier nicht unbedingt notwendig ist, die Marker, die in einem Bild aktiviert werden, bzw. die Art der Aktivierung der Marker zur Laufzeit dynamisch anzupassen. Vielmehr ist es ausreichend, vorab eine bestimmte Abfolge und/ oder Art der Aktivierung der Marker über die Laufzeit zu definieren, so dass basierend hierauf im Ergebnis eine möglichst effiziente globale rekursive Identifizierung der Marker möglich ist.

Hierzu können beispielweise mehrere Marker innerhalb einer sich wiederholenden Anzahl an Bildfolgen durch Variation ihrer Leuchtdauer, Farbe und/ oder Leuchtintensität derart mit einem insbesondere vordefinierten individuellen Ansteuerungsmuster angesteuert werden, das sobald einer dieser Marker über eine vorbestimmte Anzahl an aufeinanderfolgenden Bildern lokal rekursiv identifiziert wurde, dieser auch lokal rekursiv identifiziert ist, ohne dass eine global nicht-rekursive Identifikation notwendig ist. Dieses Verfahren entspricht dem eingangs beschriebenen Verfahren, bei dem direkt eine global rekursive Identifikation stattfindet, ohne dass eine globale nicht-rekursive Identifikation durchgeführt werden muss. Dies kann beispielsweise durch einen Code geschehen oder durch eine vordefinierte Abfolge des Aktivierens eines Markers, wobei bei der Interpretation der aufgenommenen Bilder des Markers dann bekannt sein muss, an welchem Punkt der Abfolge der Aktivierung des Markers sich dieser gerade befindet. Dies kann beispielsweise durch Definition des Startpunktes einer solchen Abfolge geschehen.

Eine derartige Bildfolge, die sich wiederholt, kann beispielsweise vier Bilder lang sein. Innerhalb dieser Bildfolge von vier Bildern kann die Leuchtdauer, Farbe und/ oder Leuchtintensität zweistufig angesteuert werden, so dass es möglich ist, jeden Marker, der über drei aufeinanderfolgende Bilder lokal rekursiv identifiziert wurde, auch global rekursiv zu identifizierten. Hierzu kann beispielsweise die folgende Bildfolge verwendet werden:
1 Marker A: 2121 oder 1212
2 Marker B: 2211 oder 1122
3 Marker C: 1111 oder 2222

Entsprechend ist es auch möglich, durch derartige individuelle Ansteuerungsmuster mehr als drei Marker voneinander zu unterscheiden, wobei dann entsprechend die Bildfolgen länger gewählt werden müssen, da die Anzahl der aufeinanderfolgenden Bilder, in denen der Marker lokal rekursiv identifiziert worden sein muss, um global rekursiv identifizierbar zu sein, größer ist.

In der beschriebenen Ausführungsform ist es bei der Auswertung der aufgenommenen Bilder nicht notwendig, Kenntnisse über die Leuchtdauer, Farbe und/ oder Leuchtintensität eines Markers im jeweiligen Bild zu haben. Es muss lediglich Kenntnis über die vordefinierten individuellen Aktivierungsmuster bestehen. Insofern eignet sich diese Ausführungsform des Verfahrens auch für Eingabevorrichtungen, die keine Kommunikationsverbindung zu den Markern aufweisen.

Es ist weiterhin bevorzugt, dass bei der Verwendung mehrerer Kameras diese zeitversetzt belichtet werden und die Frequenz, mit der ein Marker aktiviert wird, höher ist als die Frequenz, mit der eine Kamera belichtet wird, so dass ein Marker mehrere Kameras mit individuellen Aktivierungsmustern bedienen kann. Diese Ausführungsform macht insbesondere Sinn für die dynamische Markeridentifikation, da die Anforderungen für jeden Marker sich von Bild zu Bild zwischen den einzelnen Kameras unterscheiden können. Beispielsweise ist es möglich, dass eine Kamera einen Marker lokal rekursiv identifizieren kann, während eine andere Kamera dies nicht kann. Insofern ist es in diesem Fall sinnvoll für diejenige Kamera, die keine lokal rekursive Identifizierung durchführen kann, ein Aktivierungsmuster dieses Markers bereitzustellen, das eine globale nicht-rekursive Identifizierung zulässt. Da dies für die andere Kamera nicht notwendig ist, kann hier ein Aktivierungsmuster gewählt werden, das z. B. eine lokal rekursive Identifikation begünstigt, z. B. indem mehrere oder alle Marker aktiviert werden. Somit ist es in diesem Fall von Vorteil, mehrere Kameras mit individuellen Aktivierungsmustern bedienen zu können. Dies ist ein wesentliches Merkmal der dynamischen Markeridentifikation, weil hier für jede Kamera das Aktivierungsmuster eines Markers zur Laufzeit je nach Bedarf dieser Kamera angepasst werden kann.

In einer bevorzugten Ausführungsform werden in jedem zweiten Kamerabild alle Marker aktiviert, um bei möglichst vielen Markern ein lokal rekursives Tracking durchzuführen. In den dazwischenliegenden Bildern wird ein Marker, der zu diesem Zeitpunkt nicht global rekursiv identifizierbar ist, allein aktiviert oder derart aktiviert, dass sich seine Leuchtdauer, Leuchtintensität und/ oder Farbe von allen anderen in diesem Bild aktivierten Markern unterscheidet, so dass er global nicht-rekursiv identifizierbar ist.

Weiterhin ist es bevorzugt, dass für jedes aufgenommene Bild einer Kamera derjenige Marker oder diejenigen Marker dynamisch selektierbar sind, die für genau dieses Kamerabild aktiviert werden sollen, wobei das Aktivierungsmuster zur Laufzeit für jedes Kamerabild änderbar ist.

Es ist bevorzugt, Marker, von denen aufgrund der bekannten Lage und Geometrie der Eingabevorrichtung bekannt ist, dass sie auf dem Bild der Kamera ohnehin nicht sichtbar sind (beispielsweise weil sie sich auf der Rückseite der Eingabevorrichtung befinden, die gerade nicht sichtbar ist) in diesem aktuellen Kamerabild nicht aktiviert werden, da durch ihre Aktivierung ohnehin kein Mehrwert geschaffen würde. Dies gilt zumindest für Kamerabilder, in denen nicht ohnehin sämtliche Marker ohne eine Vorauswahl aktiviert werden.

Im Folgenden werden bevorzugte Ausführungsformen der Erfindung anhand von Figuren erläutert.

Es zeigen:
- Figuren 1 und 2: einen prinzipiellen Aufbau eines Systems zur Durchführung des erfindungsgemäßen Verfahrens,
- Figur 3: die Ansteuerung von zwei Kameras und drei Markern,
- Figur 4: eine Eingabevorrichtung, die eine Telemanipulationsroboter steuert.

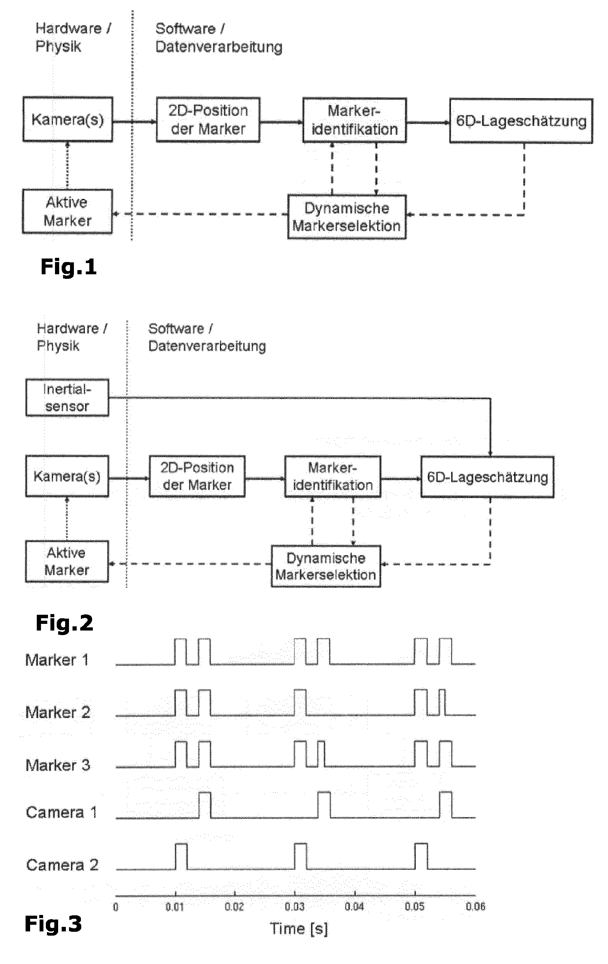

Ein Ausführungsbeispiel des erfindungsgemäßen Verfahrens ist in Figur 1 dargestellt. Eine oder mehrere Kameras nehmen Bilder auf und senden diese zu einer bildverarbeitenden Instanz, die auf der rechten Seite der Figur dargestellt ist. Im Rahmen dieser Bildverarbeitung werden die 2D-Positionen der Marker bestimmt. Die extrahierten 2D-Koordinaten werden unsortiert zu einer Markeridentifikation gesendet, welche alle Marker, bei denen es möglich ist, aufgrund der Historie eindeutig identifiziert (global rekursive Identifizierung). Eine globale nicht-rekursive Identifizierung eines Markers ist auch möglich, wenn alle aktivierten Marker bis auf diesen einen Marker eindeutig aus der Historie (d.h. lokal rekursiv) identifizierbar sind.

Die dynamische Markerselektion erhält die Lage der zu messenden Eingabevorrichtungen aus der 6D-Lageschätzung sowie die Liste der gemessenen und identifizierten Marker und der nicht identifizierten Marker aus der Markeridentifikation. Mit dieser Information werden im Rahmen der dynamischen Markerselektion die zu aktivierenden Marker ausgewählt. Eine einfache Möglichkeit hierzu ist ein Verfahren, bei dem abwechselnd immer alle Marke und jeweils nur ein Marker aktiviert werden. Aus der Menge der nicht identifizierten Marker wird ein geeigneter Marker gewählt, welcher für eine Messung als einziger aktiviert wird. Die Markeridentität der zweidimensionalen Messung wird dann zur Markeridentifikation gesendet, welche den ausgewählten Marker durch nicht rekursive Identifikation verifiziert. In der nächsten Messung werden wieder alle Marker aktiviert. Die vorher identifizierten Marker werden lokal rekursiv durch ein Tracking im 2D-Bild identifiziert, so dass sie auch global rekursiv identifiziert sind. Unter allen nicht identifizierten Markern wird wieder einen Marker für die nächste Messung ausgewählt, welcher als einziger aktiviert wird. Auch hiernach findet wieder eine Bestätigung dieser Hypothese statt. Eine direkte Zuweisung der Markeridentität durch die 6D-Lageschätzung findet nicht statt.

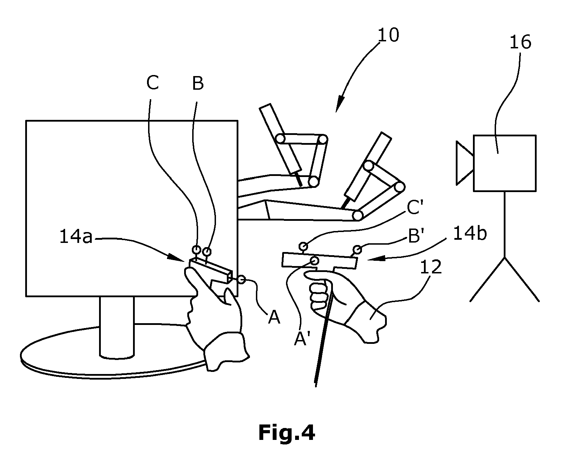

Eine Erweiterung kann gemäß Figur 4 dadurch stattfinden, dass im Rahmen der 6D-Lageschätzung Messdaten eines Intertialsensors verwendet werden.

Figur 3 zeigt ein Beispiel für eine zeitliche Abfolge der Kamera- und Markeraktivierung mit zwei Kameras und drei Markern. Die beiden Kameras werden phasenversetzt getaktet mit 50 Bildern/s (20 ms/Takt) und 2 ms Belichtungszeit. Die Marker werden für jede Kameraphase in 2 (bzw. 3) Stufen aktiviert: (a) Marker bleibt deaktiviert, (b) Marker wird für die halbe Phase (1 ms) aktiviert, (c) Marker wird die ganze Phase (2 ms) aktiviert.

Eine schematische Darstellung eines Telemanipulationsroboters 10, der durch eine Eingabevorrichtung 14a, 14b gesteuert wird, ist in Figur 4 dargestellt. Jede Eingabevorrichtung weist hierbei drei Marker A, B, C, A', B', C' auf, die durch die Kamera 16 erfasst werden. Die Eingabevorrichtungen 14a, 14b werden durch die Bedienperson 12 bewegt, so dass ihre Bewegungen auf die beiden Roboterarme des Roboters 10 übertragen werden.

## Patentansprüche

1. Verfahren zum Steuern eines Telemanipulationsroboters (10), mit den folgenden Schritten:
a) Optisches Erfassen einer durch eine Person hervorgerufenen Bewegung einer Eingabevorrichtung (14a, 14b) durch mindestens eine Kamera (16), wobei die Eingabevorrichtung (14a, 14b) mindestens drei aktive Marker A, B, C, A', B', C' aufweist;
b) Berechnen von Bewegungsbefehlen für den Telemanipulationsroboter (10) aus den gemäß Verfahrensschritt a) erfassten Bewegungen der durch die Person (12) bewegten Eingabevorrichtung (14a, 14b);
c) Übertragen der Bewegungsbefehle an den Telemanipulationsroboter (10) und Bewegen des Telemanipulationsroboters (10) gemäß den übertragenen Befehlen,
**dadurch gekennzeichnet, dass**
eine lokale rekursive Markeridentifikation durchgeführt wird, indem in mindestens einem Bild der hierauf sichtbare Marker A einem Marker A zugeordnet wird, der auf einem Bild sichtbar ist, das zu einem früheren Zeitpunkt von derselben Kamera (16) aufgenommen wurde,
eine globale Markeridentifikation durchgeführt wird, indem ein Marker A global nicht-rekursiv in einem Bild einer Kamera eindeutig identifiziert wird und/ oder der Marker A alternativ oder zusätzlich durch ein eindeutiges Ansteuerungsmuster in mehreren zeitlich hintereinander folgenden Bildern derselben Kamera global rekursiv identifiziert wird,
und eine globale rekursive Identifikation durchgeführt wird, wenn ein Marker A in mindestens zwei Bildern lokal rekursiv identifiziert wurde und in mindestens einem dieser Bilder global nicht-rekursiv und/ oder durch ein eindeutiges Ansteuerungsmuster in mehreren Bildern global rekursiv identifiziert wurde,
wobei dann dieser Marker A für alle Bilder, in denen er lokal rekursiv identifiziert wurde, als global rekursiv identifiziert gilt.

2. Verfahren nach Anspruch 1, **dadurch gekennzeichnet, dass** eine globale nicht-rekursive Identifikation eines Markers A durchgeführt wird, indem die Leuchtdauer, die Leuchtintensität und/ oder die Farbe dieses Markers A gegenüber allen anderen Markern B, C variiert wird, und/ oder indem alle anderen auf dem aktuellen Bild sichtbaren Marker B, C lokal rekursiv identifiziert werden, so dass lediglich ein einziger Marker übrig bleibt, der global nicht-rekursiv identifizierbar ist.

3. Verfahren nach Anspruch 1 oder 2, **dadurch gekennzeichnet, dass** für jedes aufgenommene Bild einer Kamera (16) derjenige Marker oder diejenigen Marker dynamisch selektierbar sind, die für genau dieses Kamerabild aktiviert werden sollen, wobei das Aktivierungsmuster zur Laufzeit für jedes Kamerabild änderbar ist.

4. Verfahren nach Anspruch 2, **dadurch gekennzeichnet, dass** eine globale nicht-rekursive Identifikation eines Markers A durchgeführt wird, indem die Leuchtdauer, Farbe und/ oder die Leuchtintensität dieses Markers A in einem Bild derart gewählt wird, dass sie sich von der Leuchtdauer, Farbe und/ oder der Leuchtintensität aller anderen auf diesem Bild sichtbaren Marker B, C, A', B', C' unterscheidet und in einem nächsten Bild ein anderer Marker B auf dieselbe Weise global nicht-rekursiv identifiziert wird, wobei bei der Auswertung eines aufgenommenen Bildes insbesondere Kenntnis darüber besteht, welcher Marker im aktuellen Bild identifiziert werden soll und durch eine lokale rekursive Identifikation in folgenden oder vorausgegangenen Bildern eine global rekursive Identifikation durchgeführt wird.

5. Verfahren nach einem der Ansprüche 2 bis 4, **dadurch gekennzeichnet, dass** ein Marker A, der durch seine einzigartige Leuchtdauer, Farbe und/ oder Leuchtintensität in einem Bild global nicht-rekursiv identifiziert wurde, so lange wie möglich lokal rekursiv identifiziert wird, so dass sich für diese Zeitdauer eine global rekursive Identifizierung dieses Markers A ergibt und die Farbe, Leuchtdauer und/ oder die Leuchtintensität dieses Markers A insbesondere erst dann gegenüber allen anderen Markern B, C, A', B', C' variiert wird, wenn dieser Marker A nicht mehr ohne Unterbrechung lokal rekursiv identifiziert werden kann.

6. Verfahren nach einem der Ansprüche 1 bis 5, **dadurch gekennzeichnet, dass**, wenn sich mehrere Marker A, B in einem Bild derart aneinander annähern, dass eine Fortführung des lokalen rekursiven Identifizierens eines dieser Marker A, der bereits global nicht-rekursiv identifiziert wurde, aufgrund einer Verwechslung mit einem anderen Marker B gefährdet ist, dieser andere Marker B deaktiviert oder derart angesteuert wird, dass eine Unterscheidung des ersten Markers A möglich ist.

7. Verfahren nach einem der Ansprüche 1 bis 6, **dadurch gekennzeichnet, dass** eine globale nicht-rekursive Identifizierung eines Markers A durchgeführt wird, indem basierend auf der bekannten Geometrie der Anordnung der Marker A, B, C, insbesondere unter Einbeziehung von Messwerten einer Inertialmesseinheit mit einem Beschleunigungssensor, Drehratensensor und /oder Kompass eine Hypothese darüber aufgestellt wird, welcher Marker gerade sichtbar ist und dieser Marker A anschließend gezielt durch eine Variation seiner Leuchtdauer, Leuchtintensität und/oder Farbe gegenüber allen anderen in diesem Bild sichtbaren Markern angesteuert wird, wobei, wenn es sich bei diesem Marker tatsächlich um den in der Hypothese vermuteten Marker handelt, diese Hypothese verifiziert ist und der Marker global nicht-rekursiv identifiziert ist.

8. Verfahren nach einem der Ansprüche 1 bis 4, **dadurch gekennzeichnet, dass** mehrere Marker A, B innerhalb einer sich wiederholenden Anzahl an Bildfolgen durch Variation ihrer Leuchtdauer, Farbe und/ oder Leuchtintensität derart mit einem insbesondere vordefinierten individuellen Ansteuerungsmuster angesteuert werden, dass sobald einer dieser Marker über eine vorbestimmte Anzahl an aufeinanderfolgenden Bildern lokal rekursiv identifiziert wurde, dieser auch global rekursiv identifiziert ist, ohne dass eine global nicht-rekursive Identifikation notwendig ist.

9. Verfahren nach Anspruch 8, **dadurch gekennzeichnet, dass** drei Marker A, B, C innerhalb einer sich wiederholenden Anzahl von vier Bildfolgen in ihrer Leuchtdauer und/ oder Leuchtintensität zweistufig angesteuert werden, gemäß dem Ansteuerungsmuster:
1 Marker A: 2121 oder 1212
2 Marker B: 2211 oder 1122
3 Marker C: 1111 oder 2222
so dass jeder Marker, der über drei aufeinanderfolgende Bilder lokal rekursiv identifiziert wurde, auch global rekursiv identifiziert ist.

10. Verfahren nach Anspruch 8 oder 9, **dadurch gekennzeichnet, dass** bei der Auswertung der aufgenommenen Bilder keine Kenntnis über die Leuchtdauer und/ oder die Leuchtintensität eines Markers im jeweiligen Bild bestehen muss und lediglich Kenntnis über die vordefinierten individuellen Aktivierungsmuster bestehen muss.

11. Verfahren nach einem der Ansprüche 1 bis 10, **dadurch gekennzeichnet, dass** bei Verwendung mehrerer Kameras (16), diese zeitversetzt belichtet werden und die Frequenz, mit der ein Marker A aktiviert wird, höher ist als die Frequenz, mit der eine Kamera (16) belichtet wird, so dass ein Marker mehrere Kameras (16) mit individuellen Aktivierungsmustern bedienen kann.

12. Verfahren nach einem der Ansprüche 1 bis 11, **dadurch gekennzeichnet, dass** in jedem zweiten Kamerabild alle Marker A, B, C, A', B', C' aktiviert werden, um bei möglichst vielen Markern ein lokal rekursives Tracking durchzuführen und in den dazwischenliegenden Bildern ein Marker A, der zu diesem Zeitpunkt nicht global rekursiv identifizierbar ist, allein aktiviert wird oder derart aktiviert ist, dass sich seine Leuchtdauer, seine Leuchtintensität und/ oder seine Farbe von allen anderen in diesem Bild aktivierten Markern B, C unterscheidet, so dass er global nicht-rekursiv identifizierbar ist.
